# EUROPEAN PATENT APPLICATION

(11) **EP 2 020 207 A1**
(43) Date of publication of application: **04.02.2009**
(21) Application number: 08169906.8
(22) Date of filing: 01.03.2006
(51) Int. Cl.: A61B 17/00

(54) **Specimen retrieval pouch and method of use**

(30) Priority: 07.03.2005 US 73536
(62) Divisional of application: 06004116.7
(71) Applicant: Tyco Healthcare Group LP, Norwalk, Connecticut 06856 (US)
(72) Inventor: Sniffin, Kevin, Danbury, CT 06810 (US)
(74) Representative: HOFFMANN EITLE

(57) **Abstract**

A specimen removal apparatus includes a pouch assembly and applicator. The pouch assembly is fabricated from a flexible membrane. The applicator includes a drawstring and an endoscopic tubular portion. When the drawstring is pulled, it closes the mouth of the pouch assembly and detaches the pouch assembly from the applicator. The pouch assembly includes a seal proximal to the mouth. A suction apparatus includes a suction tube, a tubular member, and a suction source. The suction apparatus is attached to the pouch assembly and the seal forms a substantially fluid-tight barrier around the suction tube. Suction is applied to evacuate solid and/or liquid material from the pouch assembly.

## Description

### BACKGROUND

### 1. Field of the Invention

The present disclosure relates to a surgical containment apparatus and methods for use thereof. More particularly, the present disclosure relates to a specimen retrieval pouch and method for use in minimally invasive surgical procedures.

### 2. Background of the Art

Laparoscopic and endoscopic surgical procedures are minimally invasive procedures in which operations are carried out within the body by using elongated instruments inserted through small entrance openings in the body. The initial opening in the body tissue to allow passage of the endoscopic or laparoscopic instruments to the interior of the body may be a natural passageway of the body, or it can be created by a tissue piercing instrument such as a trocar. Laparoscopic and endoscopic procedures generally require that any instrumentation inserted in the body be sealed, i.e. provisions must be made to ensure that gases do not enter or exit the body through the instrument or the entrance incision so that the surgical region of the body, e.g. the peritoneum, may be insufflated. Mechanical actuation of such instruments is for the most part constrained to the movement of the various components along a longitudinal axis with structure provided to convert longitudinal movement to lateral movement where necessary.

Because the endoscopic or laparoscopic tubes, instrumentation, and any required punctures or incisions are relatively narrow, endoscopic or laparoscopic surgery is less invasive as compared to conventional surgical procedures in which the surgeon is required to cut open large areas of body tissue. Therefore, laparoscopic or endoscopic surgery minimizes trauma to the patient and reduces patient recovery time.

Minimally invasive procedures may be used for partial or total removal of body tissue or organs from the interior of the body, e.g. nephrectomy, cholecystectomy, and other such procedures. During such procedures, it is common that a cyst, tumor, or other affected tissue or organ must be removed via the access opening in the skin, or through a cannula. Various types of entrapment devices have been disclosed to facilitate this procedure.

For example, U.S. Pat. No. 5,037,379 to Clayman et al. disclose a surgical tissue bag for percutaneously debulking tissue by morcellation. The bag includes a layer of puncture-resistant material, a layer of moisture-resistant material and a drawstring. In a disclosed method of use, the bag is placed within the body cavity, the body tissue or organ is placed within the bag, the opening of the bag is pulled through the incision in the skin leaving the distal end of the bag containing the tissue or organ within the body cavity, a morcellator is then inserted into the bag, and then the tissue or organ is debulked and suctioned out of the bag.

U.S. Pat. No. 5,074,867 to Wilk discloses a planar membrane having filaments attached to its corners. The membrane is placed within a body cavity with the filaments extending through the trocar cannula to the outside of the body. The organ or tissue to be removed is placed on the membrane and the filaments are pulled to close the membrane around the organ and draw it through the cannula, if the organ is sufficiently deformable. If the organ is not sufficiently deformable, e.g. because of the presence of gallstones, a forceps or other instrument is used to crush the stones or tissue.

### SUMMARY

The present disclosure is directed towards an apparatus for removing body tissue from the interior of the body as part of a minimally invasive surgical procedure. The apparatus includes a pouch assembly and a pouch support. The pouch assembly preferably includes a pouch and a pouch support. The pouch has an openable end and an opposed closed end. The pouch support can be attached to an applicator assembly. The pouch may have perforations to facilitate detachment of the pouch from the pouch support. The detachment can be simultaneous with the closing of the pouch in response to pulling a drawstring.

In another embodiment, the pouch assembly includes a number of sacs where each sac has a different diameter forming a staggered arrangement of the sacs. Each sac has a mouth at one end. One of the sacs, preferably the most distal sac, has a closed end thereby forming a cavity therein, while the other sacs have an orifice opposite the mouth of the sac. The pouch assembly may have a scored line on one of the sacs to facilitate detachment of the pouch assembly from the support. The pouch assembly can have a scored line extending circumferentially therearound between the locations of the spring structure and the drawstring. The pouch support can be attached to the applicator assembly. The detachment can be simultaneous with the closing of the pouch assembly in response to pulling the drawstring thread.

The sacs can be fabricated from a material selected from the group consisting of polyurethane and latex and preferably is transparent. One or more reinforced regions or bands extend circumferentially about the pouch assembly and overlap the junction between a pair of adjacent sacs.

The pouch assembly of either embodiment may include a seal formed from an elastic material that is disposed in a proximal region of the pouch assembly, preferably near the mouth. The seal is biased by the elastic material to a closed configuration and is neid in an open configuration by the spring. In preferred configurations, the closed configuration of the seal forms a substantially fluid-tight barrier about the external surface of surgical instrument such as the suction tube.

The apparatus can further include structure for resiliently opening the openable end of the pouch assembly, such as spring structure circumferentially attached to the openable end of the pouch assembly and movable between an elongated and narrow closed configuration and a rounded open configuration, the spring structure being resiliently biased to the open configuration. The spring structure, which can support the pouch assembly as well as open it, is attached to the distal end of a drive rod and is slidably movable through a tubular portion of the applicator apparatus when in the closed configuration, and resiliently moveable to its open configuration when moved outside said tubular portion. The spring structure can include two elastic prongs each having a proximal end portion having a side surface in facing relation to the side surface of the proximal end portion of the other elastic prong and fastened thereto, and each elastic prong further having a distal end portion joined to the distal end portion of the other prong by a flexible membrane, such as shrink-wrap type tubing, attached to both said end portions.

The apparatus preferably further includes at least one gaseous sealing structure, such as a coating of viscous sealing material applied to the outer surfaces of the drive rod and the drawstring.

A suction apparatus may be included as part of the removal apparatus. The suction apparatus includes a suction tube, a tubular member, and a suction source. The suction tube is configured and dimensioned to be inserted through an access device such as a trocar cannula.

One preferred method for debulking the tissue specimen is the use of the suction apparatus in combination with any of the previously disclosed embodiments of the pouch assembly. The suction is communicated from the suction source through the tubular member, the suction tube, and ultimately to the pouch assembly through the trocar cannula. By applying a controlled amount of suction, the suction apparatus reduces the volume of solid and/or liquid matter in the pouch assembly. After the desired amount of volume reduction, the applied suction may be reduced or eliminated. The size of the tissue specimen, the volume of liquid present in the pouch assembly, the size of the access opening, and the procedure being performed are considerations in determining the amount of suction applied from the suction apparatus. The flexible membrane of the pouch assembly collapses to conform to the tissue specimen thereby facilitating the removal of the pouch assembly and tissue specimen from the body cavity. By using this method in conjunction with the disclosed embodiments of the pouch assemblies, larger tissue specimens may be removed through trocar cannulae than with conventional pouch assemblies. The suction apparatus may further include storage structure for retaining solid and/or liquid matter removed from the pouch assembly.

### BRIEF DESCRIPTION OF THE DRAWINGS

Preferred embodiments of the present disclosure are described hereinbelow with reference to the drawings wherein:
FIG. 1A is a perspective view of a pouch assembly and a deployment apparatus according to an embodiment of the present disclosure;
FIG. 1B is a perspective view of a pouch assembly and the deployment apparatus according to another embodiment of the present disclosure;
FIG. 1C is a perspective view of the apparatus in the initial, undeployed configuration;
FIG. 2 is an elevational partially cut away view of the pouch assembly according to a first embodiment of the present disclosure;
FIG. 3 is an elevational partially cut away view of the pouch assembly according to a second embodiment of the present disclosure;
FIG. 3A is another embodiment of the pouch assembly of FIG. 3 having reinforced regions;
FIG. 3B is an alternate embodiment of the pouch assembly of FIG. 3A;

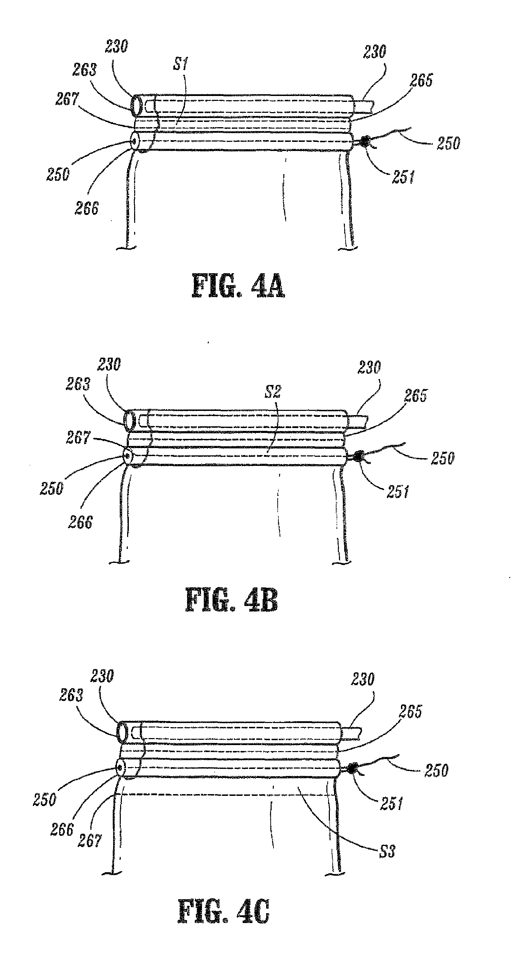

FIGS. 4A-C are elevational partially cut away views of a portion of the pouch assembly with a seal according to embodiments of the present disclosure; and
FIGS. 5A-D illustrate a method of minimizing the contents of a pouch assembly in accordance with an embodiment of the present disclosure.

### DETAILED DESCRIPTION OF PREFERRED EMBODIMENT

As used herein with reference to the present disclosure, the terms "laparoscopic" and "endoscopic" are interchangeable and refer to instruments having a relatively narrow operating portion for insertion into a cannula or a small incision in the skin, or to a surgical procedure in which such instruments are employed. Use herein of the term "laparoscopic" should not be construed to exclude "endoscopic" and use herein of the term "endoscopic" should not be construed to exclude "laparoscopic." To the contrary, It is believed that the present disclosure may find use in any procedure where access to the interior of the body is limited to a relatively small incision, with or without the use of a cannula, including, but not limited to, laparoscopic procedures.

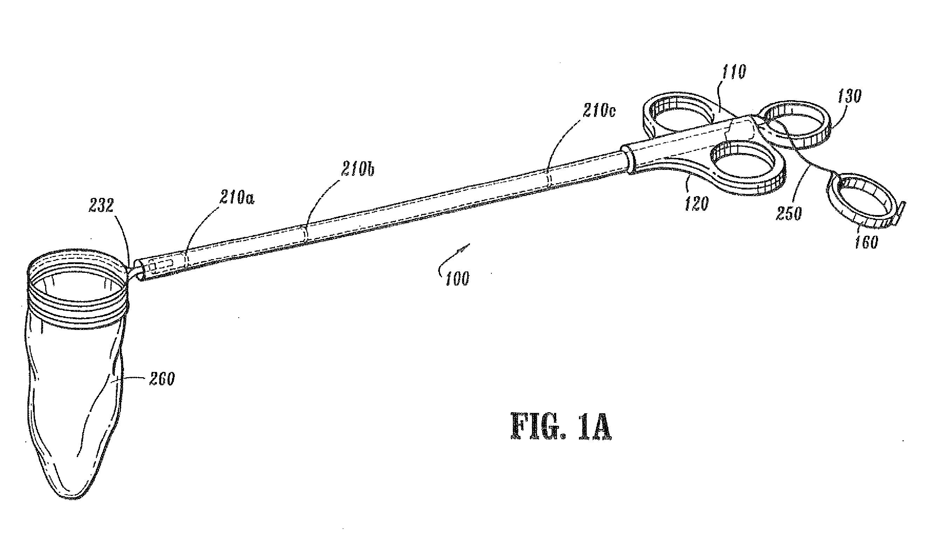

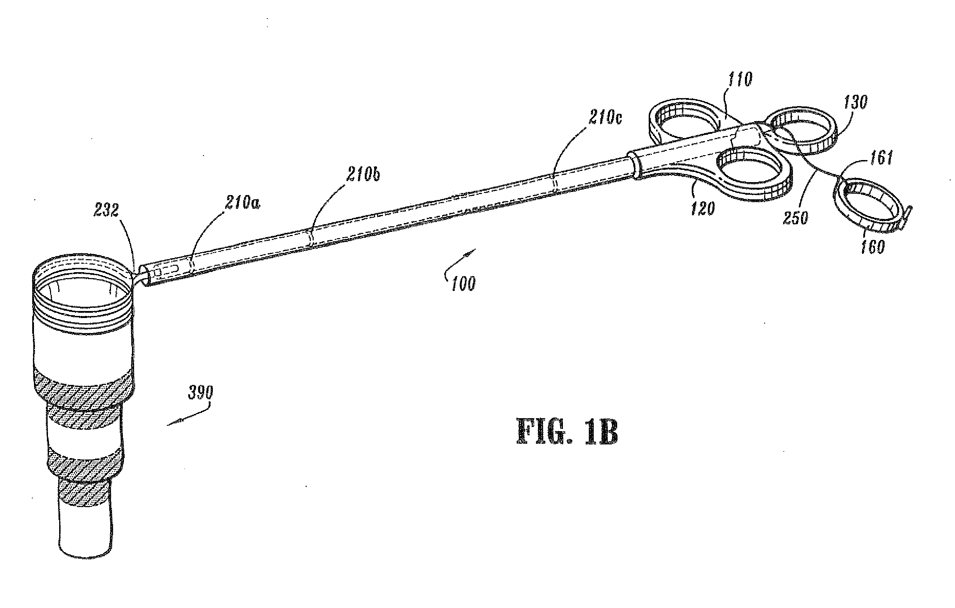

An applicator assembly 100 is illustrated in FIGS. 1A-C. As shown in FIG. 1A, the applicator assembly 100 includes a first embodiment of a removal pouch assembly 260, while FIG. 1B illustrates the applicator assembly 100 with a second embodiment of a removal pouch assembly 390. An applicator assembly suitable for use in conjunction with either removal pouch assembly is disclosed in U.S. Patent No. 5,647,372 to Tovey et al. and in U.S. Patent No. 5,465,731 to Bell et al. and the entire contents of each is hereby incorporated by reference in their entirety.

The applicator assembly 100 includes an elongated tube 180 which is of such dimensions to be insertable through an access device, such as a trocar cannula for endoscopic or laparoscopic procedures. The tube 180 is of such diameter as to permit it to be slidably disposed through a trocar cannula for use in endoscopic or laparoscopic operations, and is generally between about 0.25 inches to 0.50 inches in diameter, and about 10 inches to about 15 inches long, although other dimensions may also be used if appropriate to the operation being performed. Tube 180 slidably houses the drive rod 190 and, when undeployed, a spring 230 and pouch assembly 260. In the initial, unused condition, pouch assembly 260 will be rolled up and the spring, including spring portions 231 and 232, will be relatively straight and positioned within tube 180. When the drive rod 190 is advanced, the spring 230 connected thereto will exit the distal end of tube 180 and resiliently pop open, thereby deploying and opening pouch assembly 260. Tube 180 is preferably from a metal such as stainless steel and is preferably coated with a shrink-wrap plastic such as shrinkable polyethylene fiberglass, or polyvinyl chloride of a grade suitable for use in surgical procedures.

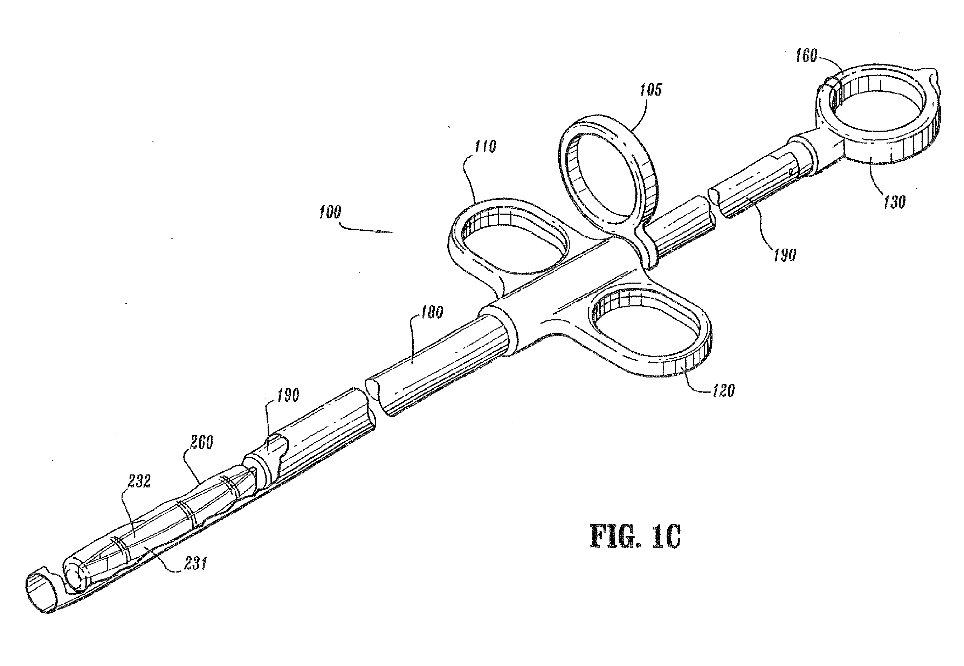

The applicator assembly 100 includes a drive rod or bar 190 that is an elongated generally cylindrical member slidably disposed through the bore of tube 180. A distal end of the drive rod 190 is attached to the pouch assembly 260 to move the pouch assembly 260 from a non-deployed position contained within the outer tube 180 (as shown in FIG. 1C) to a deployed position distal to the outer tube 180, (as shown in FIG. 1A). The drive rod 190 also includes O-rings 210a, 210b, and 210c. The O-rings help maintain a gaseous seal and/or help to maintain the drawstring in place while permitting sliding movement of the drive rod 190 through tube 180.

The drive rod 190 is preferably fabricated from a strong polymeric material. A material suitable for fabricating the drive rod 190 is polycarbonate plastic with 20% glass fiber filler. If gamma sterilization is desired, this material has the additional advantage of being gamma stable. Other materials suitable for the purposes discussed herein may also be used. To maintain a gaseous seal within the instrument, close tolerances are observed. The outer diameter of the drive rod 190 is slightly less than the inner diameter of the tube 180 through which it slides longitudinally. Additionally, the drive rod 190 is preferably coated with a biocompatible lubricant as a viscous sealing material to insure that no gases exit or enter the body through the seal when the operation site (e.g. the peritoneum or other body cavity) is insufflated. Any biocompatible lubricant that will operate as a viscous sealing material may be used, but if gamma sterilization is desired the biocompatible lubricant chosen should be gamma stable. A locking tab 105 is included to prevent premature actuation of the instrument during shipping. The locking tab includes snap fit engagement structure to engage a slot of the drive rod 190. When thus engaged, the drive rod 190 cannot be pushed distally beyond the point where the locking tab 105 engages the proximal end of handle portions 110, 120. To actuate the instrument the surgeon must first disengage the locking tab by pulling it off the instrument.

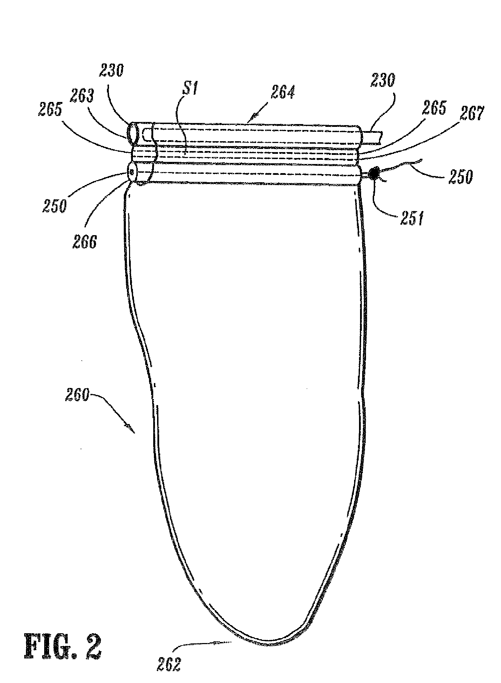

In addition, the applicator assembly 100 includes a finger loop 130 for engagement by a user's finger. One end of a drawstring 250 is attached to the finger loop 130, as shown in FIGS. 1A and 1B while an opposing end of the drawstring 250 is attached to the pouch assembly 260 (see FIG. 2).

Referring now to FIG. 2, the removal pouch assembly 260 includes a flexible film or sheet preferably formed from a substantially transparent polymeric material. One preferred material is polyurethane sheet, although other biocompatible materials capable of forming a flexible membrane, such as latex, may be used. It is also preferred that the material selected be between about 0.001 to about 0.005 inches in thickness, although other ranges of thickness may be used as appropriate. Preferably, the material is transparent to permit viewing the contents of the pouch assembly 260. In a preferred configuration, the pouch assembly 260 is formed from an aromatic polyester type thermoplastic polyurethane such as Dureflex®, a product of Deerfield Urethane, Inc. in Whately, Massachusetts. In addition, the sac material should be impervious to penetration by cancer cells.

The pouch assembly 260 may be of any dimensions suitable for the purpose of organ entrapment or removal. In the present embodiment, the pouch assembly 260 has a diameter of from about 1.5 inches to about 6.0 inches, a depth of from about 2 inches to about 10 inches, and has a cubic capacity of up to about 2.0 liters of water, depending upon the dimensions of the pouch assembly 260. Pouch assembly 260 includes a closed distal end portion 262 and an openable and closable end portion or mouth 264.

The pouch assembly 260 may alternatively include a circumferential concave portion 263 in the vicinity of the open proximal end portion or mouth 264, for facilitating rolling and placement of the pouch assembly 260 within the elongated tube 180 (See FIG. 1C). The open proximal end portion or mouth 264 is defined by a proximal (upper) circumferential tubular portion or sleeve 263, and a distal (lower) circumferential tubular portion or sleeve 266, which are spaced apart from each other.

The pouch assembly 260 possesses a linear portion 265 weakened by perforation or, more preferably, scoring, which extends circumferentially around the mouth 264 of the pouch assembly between the proximal and distal sleeves 263 and 266, respectively. The scored line 265 may be created by induction heating to create a linear portion having thickness less than that of the original material to facilitate tearing of the material along the scored line 265.

In a preferred configuration, the pouch assembly 260 includes a seal S1 formed from an elastic material. The scored line 265 defines a proximal (upper) boundary of the seal S1. The height of the seal S1 is defined between the scored line 265 and a distal (lower) boundary 267. The seal S1 has an open configuration and a closed configuration where the elastic material biases the seal S 1 to its closed configuration. As the spring exits the distal end of the applicator assembly 100, it expands to open the pouch assembly 260. The expansion of the spring 230 overcomes the bias of the elastic material to simultaneously open the mouth 264 and move the seal S 1 to its open configuration.

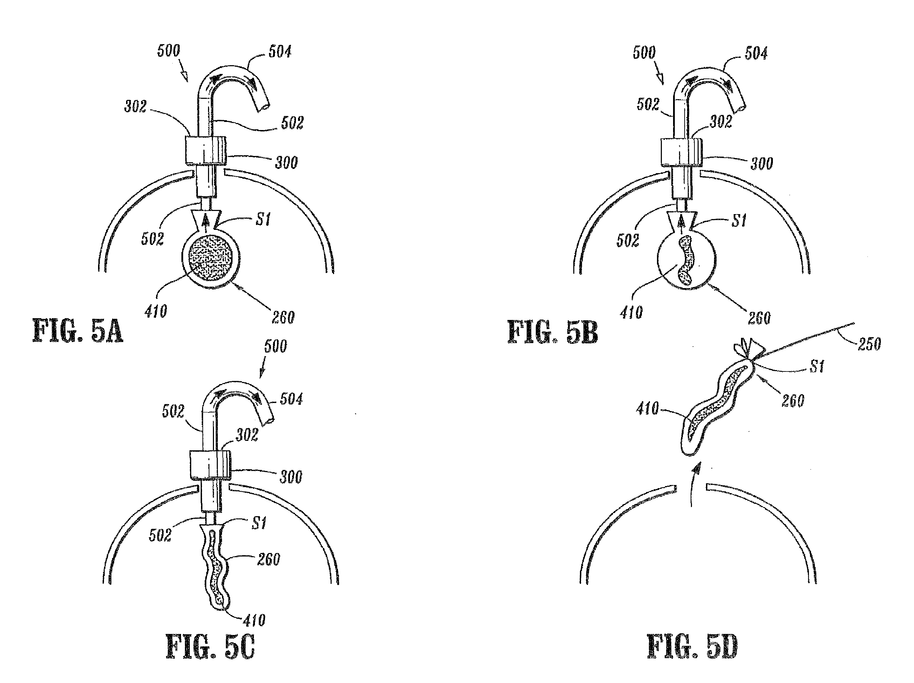

In its open configuration, the seal S 1 has substantially the same diameter as the mouth 264. It is preferred that the dimensions of the seal S1, the selected elastic material, and the pouch assembly 260 cooperate with each other such that the seal S1 will form a substantially fluid-tight barrier around a suitably sized surgical instrument (i.e. a suction tube or a morcellator) as illustrated in FIGS. 5A-C. The selected surgical instrument is configured and dimensioned to access an interior portion of a body through a suitably sized access device. It is preferred that the access device has a diameter of 10mm or 15mm, although other suitable diameters are contemplated. By forming a fluid-tight barrier around the surgical instrument, the introduction or escape of insufflation or other gases is minimized. In addition, the fluid-tight barrier minimizes the unwanted entry or escape of solids or liquids from the pouch assembly 260.

The proximal sleeve 263 is adapted to receive the spring 230, described below. The distal sleeve 266 is adapted to receive the drawstring 250. The scored line 265 is adapted to tear when the drawstring 250 is pulled with sufficient force to close the mouth 264 of the bag distal to the scored line 265, thereby providing fast detachment of pouch assembly 260 from the spring 230 simultaneously with closure of mouth 264. Clearly, alternative structures also can be utilized to detach the pouch assembly 260 from the spring 230, such as by pulling with a grasper or by cutting with a scissors. Simultaneous with the separation of the pouch assembly 260 from the spring 230, the bias of the elastic material of the seal S1 causes the seal S1 to move from its open configuration to its closed configuration.

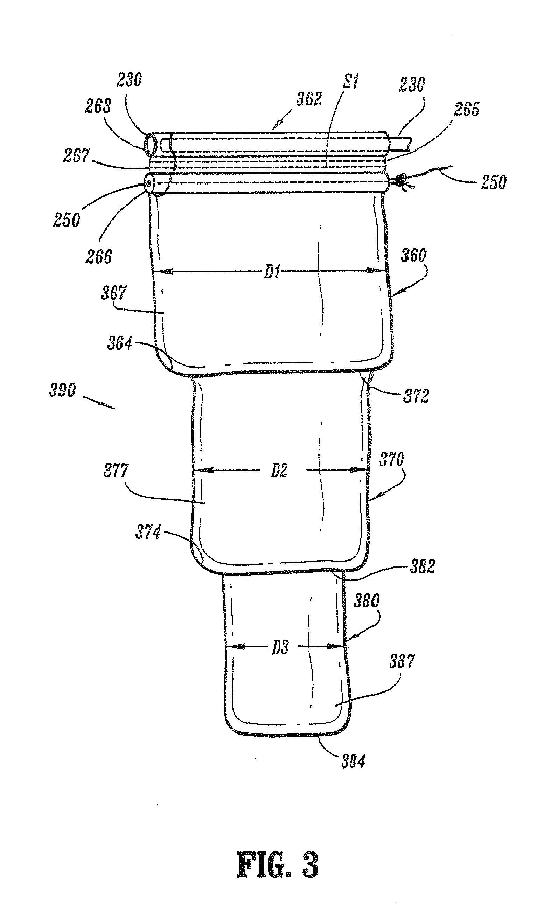

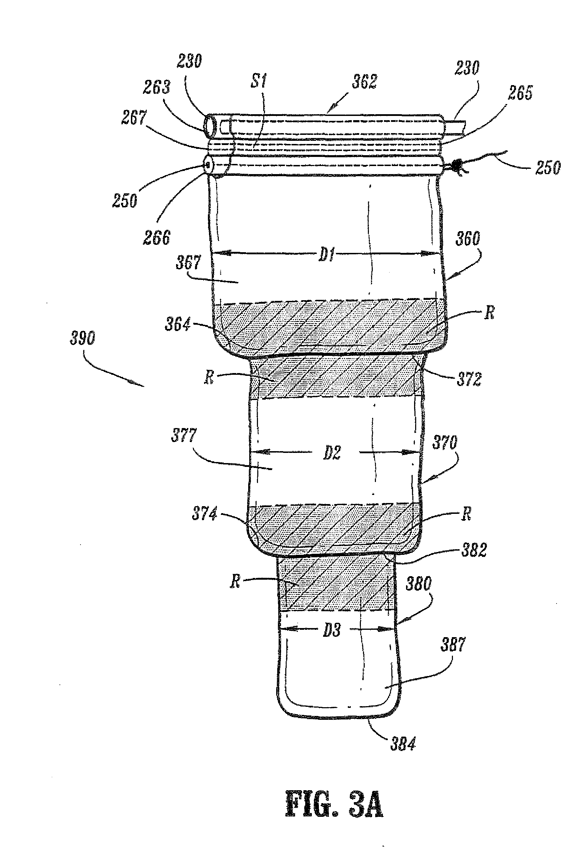

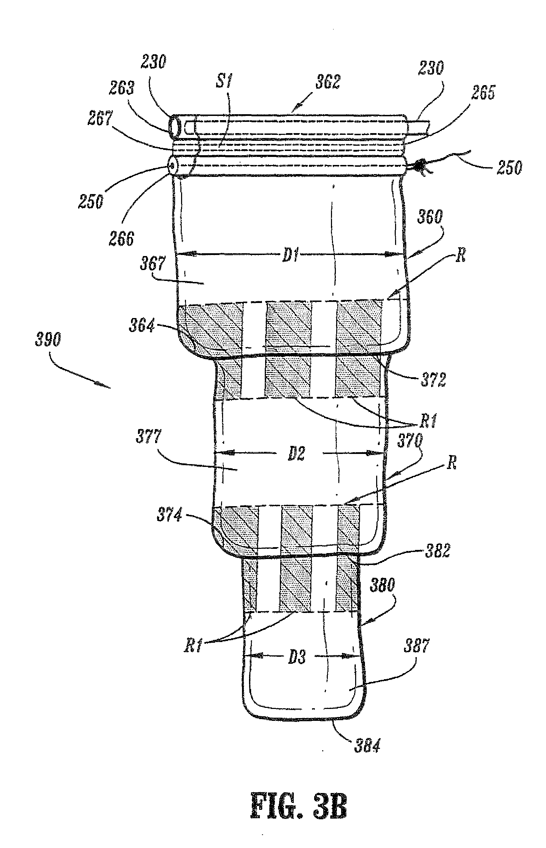

Referring to FIG. 3, an alternate embodiment of the removal pouch assembly or pouch assembly 390 includes a first sac 360, a second sac 370, and a third sac 380. Each sac is formed from a suitable material as discussed in the embodiment of FIG. 2. In addition, the pouch assembly 390 may be of any dimensions suitable for the purpose of organ entrapment or removal as discussed in the embodiment of FIG. 2.

The first sac 360 includes a mouth 362 and an orifice 364 at opposing ends defining a throat 367 therebetween. Preferably, the throat 367 has a diameter D1 that is substantially uniform from the mouth 362 to the orifice 364. Alternately, the first sac 360 may be tapered from the mouth 362 to the orifice 364 forming a frustoconical or an inverted frustoconical shape. In addition, other shapes and configurations of the sac are contemplated. The mouth 362 has an open and a closed configuration while the orifice 364 only has an open configuration.

In particular, the first sac 360 possesses a linear portion weakened by perforation or, more preferably, scoring, which extends circumferentially around the mouth 362 of the first sac 360 between proximal and distal sleeves 263 and 266, respectively. A scored line 265 may be created by induction heating to create a linear portion having thickness less than that of the original material to facilitate tearing of the material along the scored line 265.

Similar to the embodiment illustrated in FIG. 2, the pouch assembly 390 is adapted to be attached to the spring 230 using the distal sleeve 266 and includes substantially identical structures for the attachment and separation of the pouch assembly 390 of the previous embodiment with the resulting advantages discussed previously.

Still referring to FIG. 3, the second sac 370 has a mouth 372 and an orifice 374 at opposing ends defining a throat 377 therebetween. Similar to the first sac 360, the second sac 370 has a diameter D2 that is substantially uniform from the mouth 372 to the orifice 374. Alternately, the second sac 370 may be tapered from the mouth 372 to the orifice 374 forming a frustoconical or an inverted frustoconical shape. In addition, other shapes and configurations of the sac are contemplated. The mouth 372 is open and in communication with the throat 367 of the first sac 360. It is preferred that the diameter D2 or the diameter of the mouth 372 is less than the diameter D1 or the diameter of the orifice 364. Configured thusly, the throats 367, 377 of the first and second sacs 360, 370, respectively, are in fluid communication with each other and form a staggered arrangement of the sacs included in the pouch assembly 390.

The third sac 380 includes a mouth 382 and a base 384 at opposing ends. The mouth 382 is open while the base 384 is closed defining a cavity 387 therein. The cavity has a diameter D3 that is preferably uniform throughout. Alternately, the third sac 380 may be tapered from the mouth 382 to the base 384 forming a frustoconical or an inverted frustoconical shape. In addition, other shapes and configurations of the sac are contemplated. The mouth 382 is in fluid communication with the throat 377 of the second sac 370. It is preferred that the diameter D3 or the diameter of the mouth 382 is less than the diameter D2 or the diameter of the orifice 374. In this configuration, the throats 367, 377, and 387 are in fluid communication with one another to form a staggered arrangement of the pouch assembly 390.

Referring now to FIG. 3A, an alternate embodiment of the pouch assembly 390 is disclosed. The pouch assembly 390 according to this embodiment includes the same or similar components as the embodiment shown in FIG. 3 and further includes a reinforced band or region R. The reinforced region R overlaps the junction between an orifice and a mouth of a pair of adjacent sacs (e.g. orifice 364 of sac 360 and mouth 372 of sac 370). The dimensions (i.e. thickness and/or height) of the reinforced region R may be influenced by a number of factors including, but not limited to, dimensions of the pouch assembly 390, dimensions of the adjoining sacs, and the task being performed. The reinforced region R improves the overall rigidity of the pouch assembly 390 and helps maintain the staggered or stepped shape of the pouch assembly 390. Additionally, the reinforced regions R improve the strength of the joint between the adjacent sacs thereby minimizing the possibility that the sacs will separate during a surgical procedure or increasing the size and/or mass of the tissue sample to be collected.

In preferred embodiments, the reinforced region R extends circumferentially about the pouch assembly 390 although it is contemplated that it may only extend for a portion of a circumference of the pouch assembly 390. Alternatively, as illustrated in FIG. 3B, the reinforced region R may include a plurality of reinforced sections R1 that are circumferentially spaced apart forming gaps therebetween. It is preferred that each reinforced section R1 have substantially identical dimensions (i.e. thickness, height, and width), although it is contemplated that the dimensions of each of the reinforced sections R1 may be varied for the same or similar reasons discussed for the embodiment of FIG. 3A. As in the previous embodiment, the reinforced region R may extend circumferentially about the pouch assembly 390 or only for a portion thereof. In either of the embodiments of FIGS. 3A or 3B, the reinforced region R may be included in some or all of the pairs of adjacent sacs.

Alternatively, the pouch assembly may only include two sacs where the second sac has a closed end opposite its mouth defining a cavity therein. In other embodiments of the disclosure, additional sacs may be included with the last or most distal sac having a closed end opposite its mouth to define a cavity therein. These alternative configurations increase the flexibility and utility of the pouch assembly 390 of the present disclosure. The pouch assembly may be formed from discrete sacs where the sacs are bonded or joined together using known methods such that each bond is substantially fluid-tight Alternatively, the pouch assembly may be monolithically formed using known methods to create the staggered arrangement of the included sacs. The pouch assembly of these alternate embodiments may include reinforced bands or regions as previously discussed.

Referring now to FIGS. 5A-D, there is illustrated a method of using the specimen retrieval apparatus of the present disclosure. A suction apparatus 500 includes a suction tube 502, a tubular member 504, and a suction source (not shown) that is connected to a trocar cannula 300 with a first end of the suction tube 502 extending through an external port 302 of the trocar cannula 300 and into the interior portion. One end of the tubular member 504 is attached to the suction source which provides the suction applied to the pouch assembly 260. In addition, the suction apparatus may include storage for liquid and/or solid matter removed or evacuated from the pouch assembly 260. The suction tube 502 may be flexible, rigid, or semi-rigid as determined by the particular application. Preferably, the external surface of the suction tube 502 forms a fluid-tight seal with an external port 302. The second end of the suction tube 502 is attached to the one end of a tubular member 504 and forms a fluid-tight seal therebetween.

After the tissue sample 410 is placed in the pouch assembly 260 using known techniques, the mouth 264 is positioned around the first end of the suction tube 502. The pouch assembly 260 is then separated from the spring 230 by pulling the drawstring 250 in a proximal direction. This attaches the pouch assembly 260 and its contents to the suction tube 502. As discussed previously with respect to FIG. 2, the pouch assembly 260 separates from the spring 230 and the seal S 1 moves to its closed configuration and forms a substantially fluid-tight barrier around the external surface of the suction tube 502 with the attendant advantages previously discussed.

After the pouch assembly 260 is attached to the suction tube 502, the suction source is actuated and applies suction to the pouch assembly 260. The applied suction is communicated through the tubular member 504 and the suction tube 502 to the pouch assembly 260. The surgical procedure being performed, the characteristics of the tissue sample 410 (i.e. size, mass, density, etc.), and the volume of any liquids present in the pouch assembly 260 may be factors in determining the amount and duration of the applied suction. As illustrated in FIG. 5B, the applied suction to the pouch assembly 260 concurrently evacuates liquids and particulate matter (i.e. portions of the tissue sample 410 or other particulate matter) from the pouch assembly 260.

Since seal S1 forms a substantially fluid-tight barrier around the external surface of the suction tube 502, as suction is applied to the pouch assembly 260 to remove or evacuate the particulate matter and/or liquid from the pouch assembly 260, the flexible membrane collapses to conform the pouch assembly 260 to the shape and configuration of the tissue sample 410 as shown in FIG. 5C. The fluid-tight barrier formed by seal S1 minimizes the introduction of insufflation gases into the pouch assembly 260 thereby allowing the applied suction to act only on the contents of the pouch assembly without negatively affecting the insufflation pressure in the interior portion.

Once the particulate matter and/or liquid have been evacuated and the pouch assembly 260 has been reduced to a desired size, the applied suction may be discontinued or reduced. By reducing the volume of the pouch assembly 260 and its contents, improved retrieval of tissue specimens is obtained (see FIG. 5D). Further still, larger tissue samples may be retrieved for a particular size of trocar cannula using the device and methods discussed hereinabove as compared to conventional devices and methods for retrieval. Although the above discusses one embodiment of the pouch assembly, the techniques discussed are equally applicable to other embodiments of the pouch assembly previously disclosed herein.

While the above description contains many specifics, these specifics should not be construed as limitations on the scope of the present disclosure, but merely as exemplifications of preferred embodiments thereof. Those skilled in the art will envision many other possible variations that are within the scope and spirit of the present disclosure.

### Items of the Disclosure

1. A surgical apparatus for removing tissue from an interior portion of a body during a surgical procedure comprising:
   an elongate tubular member having an open distal end and a bore therein;
   a pouch assembly including a pouch support and a pouch removably attached to said pouch support, said pouch having a first end movable between an open configuration and a closed configuration, and a closed second end, the pouch assembly movable between a proximal location at least partially within said bore and a distal location at least partially exterior to said bore;
   a seal formed from an elastic material and disposed in a proximal region of said pouch, said seal having an open configuration and a closed configuration, the elastic material biasing the seal to the closed configuration and the pouch support biasing the sail in the open configuration; and
   a drawstring operatively coupled to the proximal region of the pouch wherein pulling the drawstring in a proximal direction causes the pouch to separate from the pouch support.
2. The surgical apparatus of item 1, further including an apparatus insertable into the open distal end of the pouch assembly wherein suction applied to the apparatus removes at least a portion of the tissue.
3. Apparatus which comprises means for removing a tissue specimen from an interior portion of a body during a surgical procedure comprising the steps of:
   inserting a surgical apparatus having a removal pouch releasably attached thereto into the interior portion of the body;
   opening the removal pouch;
   placing the tissue specimen in the removal pouch;
   inserting a suction tube into a mouth of the removal pouch;
   closing the mouth of the removal pouch;
   vacuuming at least a portion of the tissue specimen from the removal pouch; and
   removing the removal pouch containing the tissue specimen from the interior portion.
4. The apparatus of item 3, wherein the means for closing the mouth of the pouch includes means for forming a seal between the removal pouch and the suction tube.
5. The apparatus of any one of the preceding items, wherein the seal is substantially fluid-tight.

## Claims

1. Apparatus which comprises means for removing a tissue specimen from an interior portion of a body during a surgical procedure, comprising:
a surgical apparatus having a removal pouch releasably attached thereto, which is insertable into the interior portion of the body and removable from the interior portion with the removal pouch containing a tissue specimen placed in the removal pouch, wherein a mouth of the removal pouch is openable and closable;
a suction tube insertable into the mouth of the removal pouch; and
means for vacuuming at least a portion of the tissue specimen from the removal pouch through the suction tube.

2. The apparatus of claim 1, comprising means for closing the mouth of the pouch.

3. The apparatus of claim 2, wherein the means for closing the mouth of the pouch includes means for forming a seal between the removal pouch and the suction tube.

4. The apparatus of claim 3, wherein the seal is substantially fluid-tight.

5. The apparatus of claim 2, 3 or 4, wherein the means for closing comprises
a drawstring operatively coupled to a mouth region of the pouch, wherein pulling the drawstring in a proximal direction causes the pouch to separate from a pouch support.

6. The apparatus of claim 3 or 4, or claim 5 as dependent on claim 3 or 4, wherein the means for forming a seal comprises
a seal formed from an elastic material and disposed in a mouth region of said pouch, said seal having an open configuration and a closed configuration, the elastic material biasing the seal to the closed configuration, wherein the surgical apparatus comprises a pouch support biasing the seal in the open configuration.

7. The apparatus of any one of the preceding claims, wherein the surgical apparatus comprises
an elongate tubular member having an open distal end and a bore therein; and
a pouch assembly including a pouch support and the pouch removably attached to said pouch support, said pouch having a first end movable between an open configuration and a closed configuration, and a closed second end, the pouch assembly movable between a proximal location at least partially within said bore and a distal location at least partially exterior to said bore.

8. The apparatus of claim 7 as dependent on claim 5, wherein the pouch has perforations to facilitate detachment of the pouch from the pouch support such that the detachment is simultaneous with the closing of the pouch in response to pulling the drawstring.

9. The apparatus of claim 8, wherein the perforations are a scored line extending circumferentially around the pouch between the locations of the pouch support and the drawstring.

10. The apparatus of claim 6 or claim 7, 8 or 9 as dependent on claim 6, wherein the closed configuration of the seal forms a substantially fluid-tight barrier about the external surface of the suction tube.
